# EUROPEAN PATENT APPLICATION

(11) **EP 4 238 488 A1**
(43) Date of publication of application: **06.09.2023**
(21) Application number: 21885585.6
(22) Date of filing: 30.04.2021
(51) Int. Cl.: A61B 5/00, A61B 5/01

(54) **MIRROR SYSTEM AND AIRCRAFT LAVATORY UNIT**

(30) Priority: 29.10.2020 JP 2020181381
(71) Applicant: The Yokohama Rubber Co., Ltd., Tokyo 105-8685 (JP)
(72) Inventor: MURAYAMA, Hiroshi, Hiratsuka-shi, Kanagawa 254-8601 (JP)
(74) Representative: Dilg, Haeusler, Schindelmann Patentanwaltsgesellschaft mbH
(86) International application number: PCT/JP2021/017202
(87) International publication number: WO 2022/091461

(57) **Abstract**

To monitor the body temperature of a user (U) over a long period of time without causing the user and an attendant to perform a troublesome operation. A mirror system (10) includes a mirror (12) that reflects the user (U). An infrared camera (14) serving as a temperature detection unit detects the body surface temperature of the user (U) located around the mirror (12). A body temperature estimation unit (300) estimates the body temperature of the user (U) based on the body surface temperature. When the body temperature of the user (U) is equal to or higher than a predetermined temperature, a notification unit (302) makes a notification of high body temperature warning information indicating that the body temperature of the user (U) is equal to or higher than the predetermined temperature.

## Description

### Technical Field

The present invention relates to a mirror system provided with a mirror for reflecting a user.

### Background Art

In the related art, a technique for measuring the body temperature of a user in a non-contact manner using an infrared sensor has been developed.

For example, in the following Patent Document 1, an infrared thermometer includes an infrared sensor for detecting the intensity of infrared rays from a closed measurement portion (an ear, a mouth, an armpit) of a living body, and the infrared thermometer is configured to measure a stable body temperature (equilibrium body temperature) in a short time without being affected by an outside air temperature or a measurement state (the state of a probe portion at a measurement site) by predicting and calculating an equilibrium body temperature with a CPU based on the rise rate of measured body temperatures obtained from time-dependent infrared detection signals from the infrared sensor and displaying the equilibrium body temperature on a display.

### Citation List

### Patent Literature

Patent Document 1: JP 2001-218742 A

### Summary of Invention

### Technical Problem

In recent years, as countermeasures against infectious diseases, when it is necessary for an unspecified number of visitors to stay in the same space for a long time, each visitor is checked for his/her body temperature before entering the space, and a visitor having a high body temperature may be urged to refrain from entering the space.

However, there is a possibility that physical condition becomes worse and body temperature becomes high after the entry to the space, and thus it is preferable that body temperature can be checked on an ongoing basis if the time of staying in the space is long. On the other hand, for example, when an attendant (a person who provides services in the space, including a crew in a moving body) makes the rounds to measure the body temperature of each visitor at predetermined time intervals, the services of the attendant are increased and the visitors are inconvenienced.

The present invention has been made in view of the above circumstances, and an object of the present invention is to monitor a body temperature of a user over a long period of time without causing the user and an attendant to perform a troublesome operation.

### Solution to Problem

In order to achieve the above-described object, one embodiment of the present invention is a mirror system provided with a mirror for reflecting a user, and the mirror system includes: a temperature detection unit that detects a body surface temperature of the user located around the mirror; a body temperature estimation unit that estimates a body temperature of the user based on the body surface temperature; and a notification unit that makes a notification of high body temperature warning information when the body temperature of the user is equal to or higher than a predetermined temperature, the high body temperature warning information indicating that the body temperature of the user is equal to or higher than the predetermined temperature.

### Advantageous Effects of Invention

According to the one embodiment of the present invention, since the body temperature of the user is automatically measured when the mirror is in use, the body temperature can be measured without causing a trouble to the user and the attendant, which is advantageous in continuously monitoring body temperature particularly when a large number of persons stay in one place for a long time.

In addition, since body temperature is automatically measured by a device without human intervention, it is possible to suppress an unnecessary increase in the number of infected persons in a case where the user is infected with any infectious disease.

Further, since body temperature is measured regardless of the intention of the user, for example, even in a case where the user presents few subjective symptoms in association with a fever, it is possible to provide care before the physical condition of the user suddenly becomes worse, which is advantageous in suppressing the worsening of the symptoms.

### Brief Description of Drawings

FIG. 1 is a block diagram illustrating a configuration of a mirror system according to a first embodiment.
FIG. 2 is a diagram schematically illustrating a method of installing an infrared camera.
FIG. 3 is a diagram schematically illustrating a method of installing an infrared camera.
FIG. 4 is a flowchart illustrating a processing procedure by the mirror system.
FIG. 5 is a block diagram illustrating a configuration of a mirror system according to a second embodiment.
FIG. 6 is a diagram schematically illustrating a method of installing a visible light camera.
FIG. 7 is a diagram schematically illustrating a method of installing a visible light camera.

### Description of Embodiments

### First Embodiment

Preferred embodiments of the mirror system according to the present invention will be described in detail below with reference to the accompanying drawings.

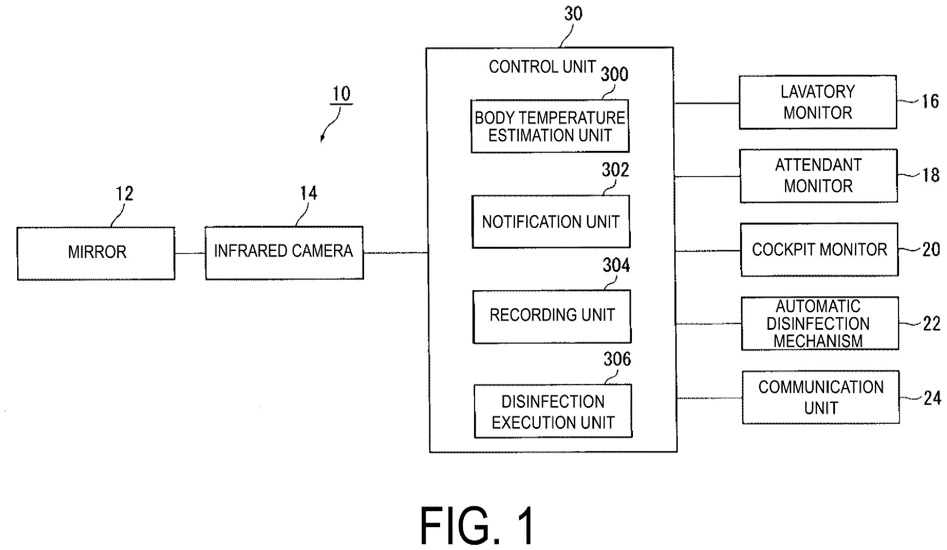

FIG. 1 is a block diagram illustrating a configuration of a mirror system according to a first embodiment.

In the first embodiment, an example in which an infrared camera 14 is used as a temperature detection unit will be described.

A mirror system 10 is provided, for example, in a moving body used by an unspecified number of passengers. The moving body is, for example, a vehicle such as an aircraft, a bullet train, or a cruise ship on which a user is assumed to board for such a long time that the user is assumed to use a lavatory. Alternatively, the mirror system 10 may be provided, for example, in a lavatory in an airport, a station, or a department store used by an unspecified number of users.

In the present embodiment, the mirror system 10 is provided in an aircraft lavatory unit.

The aircraft lavatory unit includes a toilet bowl, a hand washing sink, and the like housed in a slightly larger area than a normal toilet stall. A mirror 12 in the aircraft lavatory unit is typically provided on a wall surface adjacent to the hand washing sink. The aircraft lavatory unit has a relatively small area, and the distance between the mirror 12 and a user is small. Thus, body temperature measurement to be described later can be performed with higher accuracy.

The mirror system 10 includes the mirror 12, the infrared camera 14, a lavatory monitor 16, an attendant monitor 18, a cockpit monitor 20, an automatic disinfection mechanism 22, a communication unit 24, and a control unit 30.

The mirror 12 reflects a user U (in the present embodiment, a passenger who has entered the lavatory).

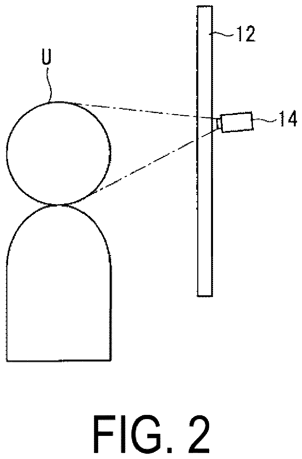

In the present embodiment, for example, as illustrated in FIG. 2, the mirror 12 is sized to reflect the face to the upper body of the user U when the user U comes to a normal mirror use position (for example, in front of the hand washing sink when the mirror 12 is provided at a hand washing place).

The infrared camera 14 functions as a temperature detection unit that detects the body surface temperature of the user U located around the mirror 12.

The infrared camera 14 detects infrared rays emitted from an object. Since the amount of infrared rays emitted from an object surface is proportional to the temperature of the object surface, the body surface temperature of the user U can be two-dimensionally detected by an image of the infrared camera 14 whose image capturing range includes the user U.

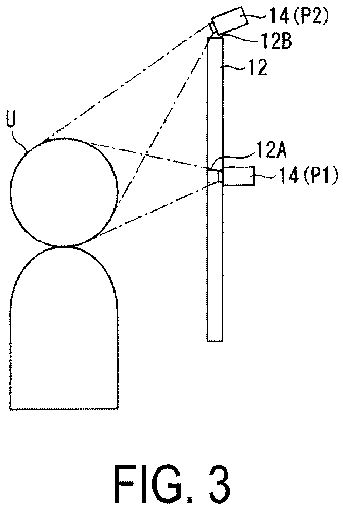

FIGS. 2 and 3 are diagrams schematically illustrating a method of installing the infrared camera.

FIG. 2 illustrates a method in which the mirror 12 is a half mirror. In this case, the infrared camera 14 is disposed, for example, at a substantially central position on the back surface side of the mirror 12 (a position where the face of the user U is highly likely to be located on the front surface side of the mirror 12). The infrared camera 14 captures infrared rays transmitted to the back surface side of the mirror 12.

FIG. 3 illustrates a method in which the mirror 12 is a total reflection mirror.

In this case, for example, a notch 12A is provided at a position of the mirror 12 where the face of the user U is highly likely to be located, and a lens of the infrared camera 14 is exposed through the notch 12A to perform image capturing. That is, the infrared camera 14 is disposed at a position P1.

In addition, for example, the infrared camera 14 may be disposed along an upper portion 12B (or a lower side) of the mirror 12, and image capturing may be performed after an image capturing direction is adjusted to a direction in which the face of the user U is highly likely to be located. That is, the infrared camera 14 is disposed at a position P2.

Returning to the description of FIG. 1, the lavatory monitor 16 is a monitor provided inside the lavatory in which the mirror 12 is installed, and presents information to the user U in the lavatory. The lavatory monitor 16 may be capable of displaying arbitrary information (characters and/or images) like a display, or may be capable of displaying only predetermined information like a seat belt sign in a passenger aircraft.

The attendant monitor 18 and the cockpit monitor 20 are monitors that present information to a crew of an aircraft (moving body). More specifically, the attendant monitor 18 is provided in the vicinity of a galley which an attendant of the aircraft enters and exits or an attendant seat, and presents information to the attendant. The cockpit monitor 20 is provided in the vicinity of a cockpit of the aircraft and presents information to a pilot. Similarly to the lavatory monitor 16, the attendant monitor 18 and the cockpit monitor 20 may be capable of displaying arbitrary information (characters and/or images) like a display, or may be capable of displaying only predetermined information.

The automatic disinfection mechanism 22 automatically disinfects each part in the lavatory, in particular, portions that are likely to be touched by users of the lavatory including the user U. Examples of the portions that are likely to be touched by the user of the lavatory include a door knob at an entrance of the lavatory, a lock operation portion at the entrance, a toilet seat, a toilet seat cleaning operation unit, and an open and close operation portion of a wash faucet.

The automatic disinfection mechanism 22 disinfects each part in the lavatory by automatically performing, for example, irradiation of ultraviolet rays, spraying of a disinfectant, ventilation of air in the lavatory, and the like.

The communication unit 24 transmits and receives information to and from another communication device on the ground (or another aircraft in flight) by wireless communication. In the present embodiment, it is assumed that the other communication device is installed at, for example, an airport, a medical facility, or the like in a place of arrival of the aircraft.

The control unit 30 includes a CPU, a ROM in which a control program and the like are recorded and stored, a RAM as an operating area of the control program, an EEPROM that holds various types of data in a rewritable manner, and an interface unit that is an interface with a peripheral circuit or the like. The CPU executes the control program, thereby functioning as a body temperature estimation unit 300, a notification unit 302, a recording unit 304, and a disinfection execution unit 306.

The body temperature estimation unit 300 estimates the body temperature of the user U based on the body surface temperature detected by the infrared camera 14. Since a method of estimating body temperature (central temperature) based on the body surface temperature of a human body is a known technique, a detailed description thereof will be omitted.

For example, the body temperature estimation unit 300 identifies the head of the user U on an image of the infrared camera 14, and estimates the body temperature based on the body surface temperature of a specific portion of the head of the user U. The specific portion of the head is, for example, the forehead, a temple, or the like. In general, the room temperature of an aircraft is controlled to about 25°C, and the user U (human) is clearly displayed as obviously having a higher temperature than other objects on a captured image of the infrared camera 14. The head of the user U facing the mirror 12 is displayed in a substantially elliptical shape. The nose, the eyes, and the like differ in temperature from surrounding portions, and thus can be extracted as features. The body temperature estimation unit 300 estimates the position of the specific portion (the forehead or the temple) based on the features and estimates the body temperature (central temperature) of the user U based on the body surface temperature at the specific portion.

Alternatively, for example, the body temperature estimation unit 300 may extract body surface temperatures at a plurality of locations on the body surface of the user U from the image of the infrared camera 14, and may estimate the body temperature based on an average value of the body surface temperatures at the plurality of locations. This method is advantageous when there is a possibility that the body surface temperature at the forehead (specific portion) cannot be obtained, for example, because the user U has bangs or wears a hat.

When the body temperature of the user U estimated by the body temperature estimation unit 300 is equal to or higher than a predetermined temperature, the notification unit 302 makes a notification of high body temperature warning information indicating that the body temperature of the user U is equal to or higher than the predetermined temperature. The predetermined temperature may be a fixed value such as 37.5 degrees, or may be a variable value as in an example to be described later.

In addition, the notification unit 302 notifies the user U of the high body temperature warning information. That is, the notification unit 302 displays on the lavatory monitor 16 a message, an icon, or the like indicating that the body temperature of the user U is higher than a normally predicted range and the user U is likely to have a fever.

For example, upon seeing this notification, the user U can notify a crew that the user U is likely to have a fever so as to receive support such as moving to a seat where the user U can rest more safely if possible, or receiving an article such as an ice bag, a cold insulator, or a blanket that can alleviate symptoms associated with the fever. Further, in particular, it is possible to cause the user U who is not aware of bad physical condition (has no subjective symptom) to be aware that the user U himself/herself has a fever, and to take measures to prevent the worsening of the physical condition at an early stage.

In addition, when the consent of every passenger of the aircraft is obtained in advance, the body temperature of the immediately previous user of the lavatory (or the presence of high temperature warning information) may be presented to the next user of the lavatory. With such a configuration, for example, when the automatic disinfection mechanism 22 is not installed in the aircraft, the next user can use the lavatory after confirming with a crew that the lavatory has been disinfected after the immediately previous user having a fever used the lavatory.

The notification unit 302 also notifies, for example, a crew of the aircraft (moving body) of the high body temperature warning information. That is, the notification unit 302 notifies at least one of the attendant monitor 18 and the cockpit monitor 20 that the body temperature of the user U who is currently using the lavatory is equal to or higher than the predetermined temperature.

Upon seeing this notification, the crew can confirm with the user U who has used the lavatory whether or not his/her physical condition has deteriorated, measure the body temperature of the user U by using a thermometer, and/or guide the user U to a seat away from other passengers, if necessary. In addition, when the automatic disinfection mechanism 22 is not installed in the aircraft, the crew can disinfect the lavatory used by the user U. That is, it is possible to actively provide care for the user U suspected of having a fever, and take measures to minimize the influence of the user U having a fever on other passengers.

The notification unit 302 transmits the high body temperature warning information of the user U to, for example, a communication device installed at a place of arrival of the aircraft (moving body). That is, the notification unit 302 notifies, via the communication unit 24, an attendant of an airport or the like at the place of arrival that a person having a fever (the user U) is on board the aircraft.

Upon seeing this notification, the attendant can prepare in advance for the acceptance of the person having a fever including, for example, securing a medical room, requesting an ambulance, and preparing an inspection to determine whether or not the person is infected with a specific infectious disease, and can quickly provide care to the person having a fever after the arrival of the aircraft.

The recording unit 304 records the body temperature of each passenger (user U) who has used the lavatory, the use time of the lavatory (for example, entry and exit times), the position information of the aircraft at the times, and the like. At this time, an image captured by the infrared camera 14 may also be stored.

By performing such recording, for example, when it is found that the user U and/or other passengers of the aircraft are infected with any infectious disease after the aircraft arrives at the place of arrival, it is possible to confirm a timing at which the user U developed a fever, confirm the health condition of the passenger who used the lavatory next to the user U, and/or confirm the state of contact between passengers who are determined to be infected with an infectious disease, which is advantageous in preventing the spread of the infectious disease.

In addition, for example, a visible light camera capable of recording the movement state of every passenger may be installed in a cabin of the aircraft to record an image during a flight, and when it is found later that some passengers were infected with any infectious disease, a warning may be given to passengers who were present in the surroundings of the infected passengers including the movement routes of the infected passengers.

When the body temperature of the user U is equal to or higher than the predetermined temperature, the disinfection execution unit 306 performs at least one of disinfection of portions that may have been touched by the user U and ventilation around the mirror 12. In the present embodiment, when the body temperature of the user U is equal to or higher than the predetermined temperature, the disinfection execution unit 306 operates the automatic disinfection mechanism 22 to perform the disinfection and/or the ventilation inside the lavatory.

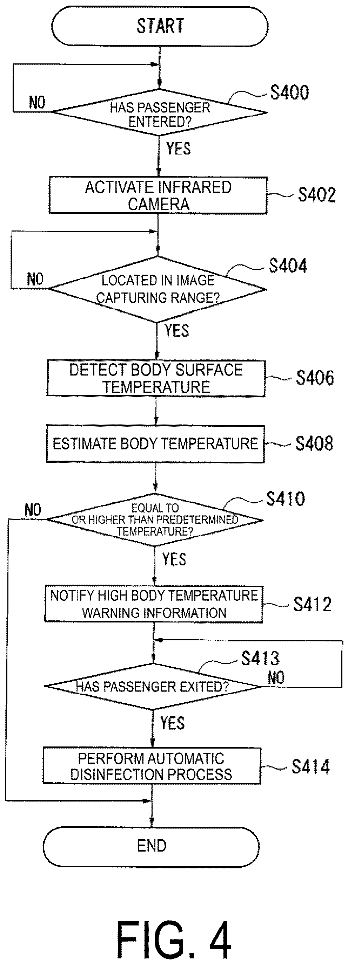

FIG. 4 is a flowchart illustrating a processing procedure by the mirror system.

When the user U (passenger) enters the lavatory (step S400: Yes), the control unit 30 activates the infrared camera 14 to start image capturing by the infrared camera 14 (step S402).

Note that, in the present embodiment, since the mirror 12 is installed in the lavatory that is a place where an entry and exit status is clear, it is economical to activate the infrared camera 14 in response to the entry and exit of the user U. However, when the mirror 12 is provided in a relatively open place (such as a deck of a bullet train), the infrared camera 14 may be always in operation.

When the user U comes in the image capturing range of the infrared camera 14 (step S404), the body temperature estimation unit 300 detects the body surface temperature of the user U from an image of the infrared camera 14 (step S406), and estimates the body temperature of the user U based on the body surface temperature (step S408).

When the temperature estimated in step S408 is lower than the predetermined temperature (step S410: No), the processing of this flowchart is ended as it is.

On the other hand, when the temperature estimated in step S408 is equal to or higher than the predetermined temperature (step S410: Yes), the notification unit 302 displays high body temperature warning information on the lavatory monitor 16, the attendant monitor 18, the cockpit monitor 20, or the like, and/or transmits the high body temperature warning information to a place of arrival via the communication unit 24, thereby notifying that the user U may have a fever (step S412).

When the user U (passenger) leaves the lavatory (step S413: Yes), the disinfection execution unit 306 operates the automatic disinfection mechanism 22 to automatically disinfect the inside of the lavatory (step S414). From a viewpoint of hygiene, automatic disinfection may be performed regardless of whether or not the user U has a fever. However, it is more practical to change the degree of disinfection operation depending on whether or not the user U has a fever (for example, only ultraviolet irradiation and ventilation are performed when the user U does not have a fever, wiping with a disinfectant is performed in addition to ultraviolet irradiation and ventilation when the user U has a fever).

When the user U leaves the lavatory, the infrared camera 14 returns to a standby state.

As described above, with the mirror system 10 according to the first embodiment, since the body temperature of the user U is automatically measured when the mirror 12 is in use, the body temperature can be measured without causing trouble to the user U and a crew, which is advantageous in continuously monitoring body temperature particularly when a large number of persons stay in one place (in the present embodiment, the aircraft) for a long time.

With the mirror system 10 according to the first embodiment, since body temperature is automatically detected by a device without human intervention, it is possible to suppress an unnecessary increase in the number of infected persons in a case where the user U is infected with any infectious disease.

With the mirror system 10 according to the first embodiment, since body temperature is measured regardless of the intention of the user, for example, even in a case where the user presents few subjective symptoms in association with a fever, it is possible to provide care before the physical condition of the user suddenly becomes worse, which is advantageous in suppressing the worsening of the symptoms.

With the mirror system 10 according to the first embodiment, since the infrared camera 14 is used as the temperature detection unit, the body surface temperature of the user U can be two-dimensionally detected, and the body temperature can be accurately estimated by using the body surface temperature of a portion suitable for body temperature estimation.

With the mirror system 10 according to the first embodiment, when the user U has a high body temperature, the inside of the lavatory is automatically disinfected, which is advantageous in reducing the work burden of a crew, maintaining the sanitary conditions of the crew, and preventing the spread of an infectious disease or the like.

With the mirror system 10 according to the first embodiment, since the body temperatures and the like of the users of the lavatory are recorded, when a passenger infected with any infectious disease in an aircraft is found, it is possible to call attention to the other passengers of the aircraft by referring to an action history, which is advantageous in preventing the spread of the infectious disease.

### Second Embodiment

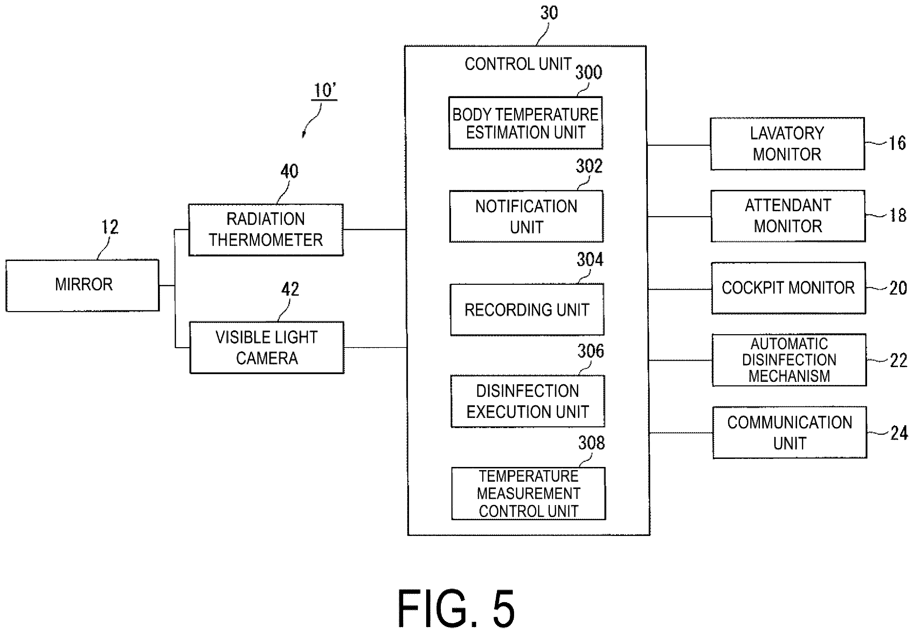

FIG. 5 is a block diagram illustrating a configuration of a mirror system according to a second embodiment.

In the second embodiment, an example in which a radiation thermometer is used as the temperature detection unit will be described.

In the second embodiment, the same components as those in the first embodiment are denoted by the same reference signs as those in the first embodiment, and detailed descriptions thereof are omitted.

A mirror system 10' illustrated in FIG. 5 includes a radiation thermometer 40 in substitution for the infrared camera 14 of the mirror system 10 according to the first embodiment, and a visible light camera 42. The control unit 30 functions as a temperature measurement control unit 308.

The radiation thermometer 40 serving as the temperature detection unit in the second embodiment detects body surface temperature based on the amount of infrared rays radiated from the body surface of the user U similarly to the infrared camera 14. However, the radiation thermometer 40 detects the temperature of one point on the body surface while the infrared camera 14 can detect the two-dimensional temperature distribution of the body surface of the user U. By using the radiation thermometer 40, the body temperature of the user U can be detected at a lower cost than the infrared camera 14.

The visible light camera 42 has an image capturing range around the mirror 12. More specifically, for example, the visible light camera 42 sets the image capturing range such that when the user U comes to a normal mirror use position, an image of a range reflected on the mirror 12, that is, an image of an range from the face to the upper body of the user U can be captured.

By using the visible light camera 42, a body temperature detection point by the radiation thermometer 40 can be identified, and the body temperature of the user U can be detected with higher accuracy. In addition, by using the visible light camera 42, for example, each user U can be more reliably identified on the data recorded in the recording unit 304.

The temperature measurement control unit 308 controls the state of body temperature detection by the radiation thermometer 40 based on an image captured by the visible light camera 42. The temperature measurement control unit 308 is provided because the radiation thermometer 40 detects the temperature of one point on the body surface as described above, and thus an appropriate detection direction needs to be set.

In the present embodiment, the temperature measurement control unit 308 functions as either a thermometer selection unit or a detection direction change unit depending on the installation mode of the radiation thermometer.

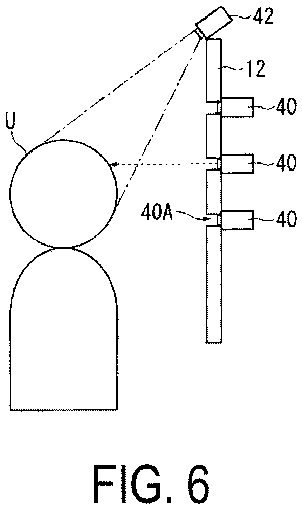

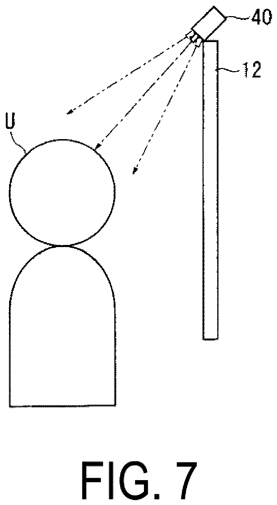

FIGS. 6 and 7 are diagrams schematically illustrating the installation method of the radiation thermometer.

FIG. 6 illustrates a method of installing a plurality of radiation thermometers 40. In this case, a plurality of notches 40A are provided in a central portion of the mirror 12 at intervals in a vertical direction, and the light receiving surfaces of the radiation thermometers 40 are exposed through these notches. In the example of FIG. 6, three radiation thermometers 40 are provided.

Although the installation position of the visible light camera 42 is arbitrary, in FIG. 6, the visible light camera 42 is disposed above the mirror 12 such that the user U standing in front of the mirror 12 is included in the image capturing range.

The temperature measurement control unit 308 detects the position of the head of the user U by performing image analysis on an image captured by the visible light camera 42, and selects the radiation thermometer 40 having a detection point closest to a specific portion (for example, the forehead) of the head of the user U among the plurality of radiation thermometers 40 installed (in the example of FIG. 6, the radiation thermometer 40 at the intermediate position is selected). Then, the body surface temperature of the user U is detected by the selected radiation thermometer 40.

That is, in the example of FIG. 6, the plurality of radiation thermometers 40 are provided around the mirror 12, and the temperature measurement control unit 308 functions as the thermometer selection unit that selects the radiation thermometer 40 to be used for detecting the body surface temperature based on an image of the visible light camera 42.

FIG. 7 illustrates a method of installing a radiation thermometer 40 having an oscillating function. In this case, for example, the radiation thermometer 40 that can move a light receiving surface vertically and horizontally is installed around the mirror 12 (above the mirror 12 in the example of FIG. 7).

Although the visible light camera 42 is not illustrated in FIG. 7, since the image capturing range of the visible light camera 42 and the temperature detection range of the radiation thermometer 40 preferably overlap with each other, the visible light camera 42 is preferably installed side by side with the radiation thermometer 40, for example.

The temperature measurement control unit 308 detects the position of the head of the user U by performing image analysis on an image captured by the visible light camera 42, and sets the light receiving direction of the radiation thermometer 40 toward a specific portion (for example, the forehead) of the head of the user U. Then, the body surface temperature of the user U is detected by the radiation thermometer 40 with the light receiving direction maintained.

That is, in the example of FIG. 7, the radiation thermometer 40 includes an oscillating mechanism that can change a temperature detection direction, and the temperature measurement control unit 308 functions as the detection direction change unit that changes the temperature detection direction of the radiation thermometer 40 based on an image of the visible light camera 42.

As described above, with the mirror system 10' according to the second embodiment, the body temperature of the user U can be detected at a lower cost than the infrared camera 14 by using the radiation thermometer 40 in addition to the effect of the mirror system 10 according to the first embodiment.

With the mirror system 10' according to the second embodiment, by using the visible light camera 42, each user U can be more reliably identified on the data recorded in the recording unit 304. This facilitates the tracking of the user U when he/she is infected with any infectious disease, and thus advantageous in preventing the spread of the infectious disease.

### Third Embodiment

In the third embodiment, a body temperature (predetermined temperature) to be determined as a high body temperature is separately set for each passenger (user U) by using machine learning or artificial intelligence (AI).

This is because, if a body temperature to be determined as a high body temperature is uniformly set as a predetermined temperature in a moving body or the like used by an unspecified number of various people, a passenger whose body temperature is lower than the predetermined temperature but whose physical condition is actually deteriorated may be overlooked, or a warning notification may be given to a passenger whose body temperature is equal to or higher than the predetermined temperature but whose physical condition is not deteriorated, making the passenger feel uneasy, or the manpower of crews may be wasted.

For example, in general, there is a tendency that children have a higher normal temperature than adults, and the normal temperature of adults decreases as the age increases. That is, the normal temperature of the user U is considered to correlate with the age of the user U.

In general, persons having a large amount of muscle or fat (a person having a large body volume) tend to have a higher normal temperature than persons having a small amount of muscle or fat. That is, the normal temperature of the user U is considered to correlate with the body type of the user U.

In general, men tend to have a larger body type and a higher normal temperature than women. That is, the normal temperature of the user U is considered to correlate with the gender of the user U.

In general, the white race tends to have a higher normal temperature than the yellow race. That is, the normal temperature of the user U is considered to correlate with the race of the user U.

These attributes of the user U can be estimated with a certain accuracy by image analysis based on an image captured by the visible light camera 42 as in the second embodiment, for example. Thus, for example, a predicted normal temperature value depending on each attribute is set in advance, and each attribute is weighted. Then, a predetermined temperature (a temperature to be determined as a high body temperature calculated by the predicted normal temperature + α) is set for each user U who has entered the lavatory, and it is determined whether or not the user U has a high body temperature based on the predetermined temperature.

Alternatively, for example, separately set predetermined temperatures and actually measured body temperatures may be compared with each other at regular intervals, and the weighting may be re-evaluated (learned) so that each predetermined temperature can be set with higher accuracy.

Factors that cause fluctuations in body temperature include an ambient temperature (the higher the ambient air temperature is, the higher the body temperature, specifically, the body surface temperature becomes) and whether or not eating or drinking has been done (in general, the body temperature rises after eating or drinking).

The ambient temperature can be obtained from an air conditioning set temperature in the aircraft and a thermometer installed in the aircraft. Regarding whether or not eating or drinking has been done, the timing at which in-flight meals are served in the aircraft is roughly determined, and the state of serving foods and drinks to each passenger can be analyzed by using an image of the visible light camera installed in the aircraft.

By further adding the factors such as the ambient temperature and whether or not eating or drinking has been done to the predetermined temperature set based on the attribute of the user U, the predetermined temperature can be set with higher accuracy.

For example, comparing a person whose body temperature rises from 37.0°C at the time of boarding the aircraft to 37.5°C, and a person whose body temperature rises from 35.8°C at the time of boarding the aircraft to 37.5°C, it can be assumed that the latter person is more likely to have an abnormal physical condition. That is, the evaluation of a detected body temperature varies depending on the body temperature history of each person.

Thus, for example, the body temperature of each passenger may be measured before boarding the aircraft, and the measured body temperature value may be associated with the face data of the passenger. Then, when the passenger enters the lavatory, his/her face may be recognized by the visible light camera 42, the measured body temperature value at the time of boarding may be referred to by using the recognized face as a key, and it may be determined whether to make a notification of the high body temperature warning information by comparing the measured body temperature value with a current body temperature.

In evaluating the current body temperature, it is preferable to consider, for example, the time elapsed from the boarding (timing of the initial body temperature measurement), and the previous body temperature value if the passenger has entered the lavatory a plurality of times.

In addition, when the body temperature measurement is performed not only at the time of boarding the aircraft, but also at the time of entry to an airport, for example, the body temperature at the time of entry to the airport may also be referred to.

That is, when the mirror system 10 (10') further includes a temperature learning unit that separately sets a predetermined temperature as a threshold value for determining whether or not to make a notification of the high body temperature warning information based on at least one of the age, the body type, the gender, the race, the ambient temperature, the eating history, and the body temperature history of the user U, the high body temperature warning information can be notified with higher accuracy, and the usefulness of the high body temperature warning information can be improved.

10 (10') Mirror system
12 Mirror
14 Infrared camera
16 Lavatory monitor
18 Attendant monitor
20 Cockpit monitor
22 Automatic disinfection mechanism
24 Communication unit
30 Control unit
300 Body temperature estimation unit
302 Notification unit
304 Recording unit
306 Disinfection execution unit
308 Temperature measurement control unit
40 Radiation thermometer
42 Visible light camera
U User

## Claims

1. A mirror system provided with a mirror for reflecting a user, the mirror system comprising:
a temperature detection unit configured to detect a body surface temperature of the user located around the mirror;
a body temperature estimation unit configured to estimate a body temperature of the user based on the body surface temperature; and
a notification unit configured to make a notification of high body temperature warning information when the body temperature of the user is equal to or higher than a predetermined temperature, the high body temperature warning information indicating that the body temperature of the user is equal to or higher than the predetermined temperature.

2. The mirror system according to claim 1, wherein the notification unit is configured to notify the user of the high body temperature warning information.

3. The mirror system according to claim 1 or 2, wherein
the mirror system is disposed in a moving body used by an unspecified number of passengers, and
the notification unit is configured to notify a crew of the moving body of the high body temperature warning information.

4. The mirror system according to claim 3, wherein the notification unit is configured to transmit the high body temperature warning information to a communication device installed at a place of arrival of the moving body.

5. The mirror system according to any one of claims 1 to 4, wherein the temperature detection unit is an infrared camera.

6. The mirror system according to claim 5, wherein the body temperature estimation unit is configured to identify the head of the user on an image of the infrared camera and estimate the body temperature based on a body surface temperature at a specific portion of the head of the user.

7. The mirror system according to claim 5, wherein the body temperature estimation unit is configured to extract body surface temperatures at a plurality of locations on a body surface of the user from an image of the infrared camera and estimate the body temperature based on an average value of the body surface temperatures at the plurality of locations.

8. The mirror system according to any one of claims 1 to 4, wherein the temperature detection unit is a radiation thermometer.

9. The mirror system according to claim 8, wherein
a plurality of radiation thermometers, each of which is identical to the radiation thermometer, are disposed around the mirror, and
the mirror system further comprises
a visible light camera configured to capture an image around the mirror, and
a thermometer selection unit configured to select, among the plurality of radiation thermometers, a radiation thermometer to be used for detecting the body surface temperature based on an image of the visible light camera.

10. The mirror system according to claim 8, wherein
the radiation thermometer comprises an oscillating mechanism that can change a temperature detection direction, and
the mirror system further comprises
a visible light camera configured to capture an image around the mirror, and
a detection direction change unit configured to change the temperature detection direction of the radiation thermometer based on an image of the visible light camera.

11. The mirror system according to any one of claims 1 to 10, further comprising a disinfection execution unit configured to perform at least one of disinfection of a portion that may have been touched by the user and ventilation around the mirror when the body temperature of the user is equal to or higher than the predetermined temperature.

12. The mirror system according to any one of claims 1 to 11, further comprising a temperature learning unit configured to separately set the predetermined temperature based on at least one of an age, a body type, a gender, a race, an ambient temperature, an eating history, and a body temperature history of the user.

13. The mirror system according to claim 12, wherein at least one of the age, the body type, the gender, and the race of the user is estimated based on an image of the user captured by a visible light camera configured to capture an image around the mirror.

14. The mirror system according to claim 12, wherein the ambient temperature is determined based on an air conditioning set temperature or a temperature obtained from a thermometer.

15. An aircraft lavatory unit comprising the mirror system described in any one of claims 1 to 14.
